(19) Europäisches Patentamt
European Patent Office
Office européen des brevets

(11) Numéro de publication: **0 023 854**
**B1**

# (12) FASCICULE DE BREVET EUROPEEN

(45) Date de publication du fascicule du brevet: **12.12.84**

(21) Numéro de dépôt: **80401053.6**

(22) Date de dépôt: **11.07.80**

(51) Int. Cl.³: **C 08 F 8/32**, C 08 J 7/14, C 08 B 37/00, A 61 F 1/00, A 61 K 31/795, A 61 K 31/725

(54) **Polymères solubles ayant des propriétés anticoagulantes, procédé de préparation et application à des médicaments à action anticoagulante.**

(30) Priorité: **20.07.79 FR 7918780**

(43) Date de publication de la demande:
**11.02.81 Bulletin 81/06**

(45) Mention de la délivrance du brevet:
**12.12.84 Bulletin 84/50**

(84) Etats contractants désignés:
**AT BE CH DE FR GB IT LI LU NL SE**

(56) Documents cités:
**DE-A-2 728 781**
**FR-A-2 066 460**
**FR-A-2 078 435**
**US-A-2 808 405**
**US-A-3 301 848**

**CHEMICAL ABSTRACTS, vol. 77, nr. 18, 30 octobre 1972, page 21, abrégé 115175t Columbus, Ohio, USA G.F. BEBIKH et al.: "Polymeric stabilizers of aging"**

**Le dossier contient des informations techniques présentées postérieurement au dépôt de la demande et ne figurant pas dans le présent fascicule.**

(73) Titulaire: **Fougnot, Christine**
**85, Rue Marcel Grandcoing**
**F-93430 Villetaneuse (FR)**
(73) Titulaire: **Jozefonvicz, Jacqueline, née Dorgebray**
**65, 2ème Avenue**
**F-60260 Lamorlaye (FR)**
(73) Titulaire: **Jozefonvicz, Marcel**
**65, 2ème Avenue**
**F-60260 Lamorlaye (FR)**

(72) Inventeur: **Fougnot, Christine**
**85, Rue Marcel Grandcoing**
**F-93430 Villetaneuse (FR)**
Inventeur: **Jozefonvicz, Jacqueline née Dorgebray**
**65, 2ème Avenue**
**F-60260 Lamorlaye (FR)**
Inventeur: **Jozefonvicz, Marcel**
**65, 2ème Avenue**
**F-60260 Lamorlaye (FR)**
Inventeur: **Mauzac, Monique, née Massat**
**46 rue Peyronnet**
**F-92200 Neuilly-sur-Seine (FR)**

(74) Mandataire: **Ores, Irène et al**
**CABINET ORES 6, Avenue de Messine**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 023 854 B1

## Description

La présente invention est relative à des polymères substitués par des groupes leur conférant des propriétés anticoagulantes et à leur procédé de préparation, aux objets constitués par et/ou comprenant lesdits polymères substitués, à leurs procédés de fabrication et à l'application desdits objects en chirurgie et en médecine, et aux compositions pharmaceutiques contenant lesdits polymères substitués.

L'héparine, agent anticoagulant naturel du sang, constitue un médicament de choix dans les affections thromboemboliques. Aussi, en vue de pallier les inconvénients de l'héparine, depuis un certain nombre d'années déjà, a-t-ou essayé de suppléer cette précieuse matière par divers produits synthétiques ou semi-synthétiques.

Ainsi, on a préconisé:—le sulfate de chondroîtine, (qui est également un polyholoside hétérogène),—le chitosane (produit de dédoublement de la chitine) préparé par la N-désacétylation totale de la chitine [HORTON et JUST "Carbohydrate Research"*29* (1973) 173—179],—le chitosane sulfaté [WOLFROM et SHENHAN, JACS (1959) 81, 1764—1766],—le sulfate d'héparane [JORPES et GARDELL "J. Biol. Chem.] 176—267 (1948)] et d'autres polysaccharides formés par la polymérisation d'une unité disaccharidique constituée par une osamine (principalement la D-glucosamine) portant un certain nombre de groupements sulfate et/ou sulfonate et par un acide uronique (généralement l'acide D-glucuronique et l'acide L-iduronique). Outre le fait que l'action analogue à celle de l'héparine ("heparin-like") de ces divers produits est moins prononcée que celle de l'héparine, leur origine naturelle est la cause (comme c'est le cas pour l'héparine) de leur variabilité et de leur hétérogénéité d'une préparation à l'autre.

Une autre série d'héparinoïdes est constituée par des produits dérivés de l'amylose (lequel, comme l'héparine a des liaisons $\alpha(1\rightarrow4)$, qui ont été étudiés, entre autres, par WOLFROM et WANG (Carbohydrate Research *18* (1971) 23—27) concernant l'amylose aminé et sulfaté et par HORTON et JUST (Carbohydrate Research *30* (1973) 349—357) concernant la conversion de l'amylose en $(1\rightarrow4)$2-amino-2-déoxy-$\alpha$D-glucopyranuronane. Les produits ainsi obtenus ont une activité héparinique faible ou pratiquement nulle (amylose aminé et sulfaté).

Certains autres polysaccharides ont été signalés pour leurs propriétés plus ou moins anticoagulantes:—le sulfate de dextrane [V. POTUZNIK J. Hyg. Epid. Microbiol. Immunol. *16*, 293 (1972)],—le polyester sulfurique de pentosane [produit vendu sous le nom de "HEMOCLAR" par les Laboratoires CLIN-MIDY], ou les dérivés de l'acide alginigue [O. LARM et collab. Carbohydr. Res. *73*, 332 (1979]. Malheureusement, aucun de ces produits ne peut être valablement comparé à l'héparine.

On a également proposé (Brevet Français 2 280 387) des dérivés sulfonés de polypeptides, à savoir des copolymères de la lysine et du tryptophane. Si ces produits provoquent effectivement un allongement très net du temps de coagulation et ont une action du même type que celle de l'héparine, ils ne peuvent pas être utilisés sur une grande échelle, car les réactions secondaires qu'ils provoquent dans l'organisme humain sont extrêmement gênantes.

En 1975, Harry P. GREGOR [Polym. Sci. Technol. U.S.A. (1975) 7, 51—56] a préconisé l'utilisation, en tant que matériaux analogues à l'héparine ("heparin-like"), de polymères et de copolymères sulfonés (en particulier des acides polystyrènesulfonique et polyéthylènesulfonique). T. BEUGELING et Collab. [J. BIOMED. MATER. RES. (1974) Vol. 8, 375—379 et BIOCOMPAT. IMPLANT. MATER, 187—192 (1976)] ont préconisé l'utilisation de polymères synthétisés à partir de polyisoprènes, dont le motif est représenté comme suit:

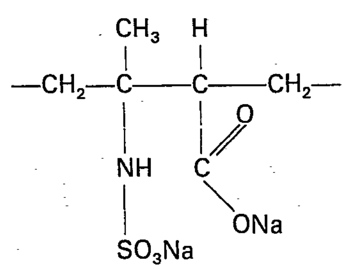

(I)

qui sont des dérivés contenant des groupes aminosulfonates et carboxylates.

Ces Auteurs ont obtenu essentiellement des dérivés solubles dont l'activité anticoagulante ne représente cependant que 12 à 15% de celle de l'héparine prise comme référence.

D'autres tentatives intéressantes ont été effectuées par greffage de la molécule d'héparine elle-même sur la surface des polymères [par exemple à l'aide de l'agent de couplage constitué par le chlorure de tridodécylméthylammonium: LEININGER et Collab. dans Trans. Amer. Soc. Artif. Int. Organs *18* 312 (1972)] ou par liaison covalente: [HOFFMAN et Collab. dans Trans. Amer. Soc. Artif. Int. Organs. *18* 10 (1972)]. Malheureusement, outre le fait que l'on ne parvient à fixer qu'une quantité relativement faible d'héparine, ces polymères "héparinisés" ne sont pas stables: la quantité fixée a tendance à s'inactiver avec le temps.

D'une manière générale, l'intérêt qu'il y aurait à pouvoir disposer d'un produit à action anti-coagulante non seulement stable, reproductible et homogène d'un lot à l'autre, est à l'origine des nombreux travaux de recherche de nouveaux anticoagulants.

Les Demandeurs ont formulé l'hypothèse selon laquelle les propriétés anti-coagulantes de l'héparine ne sont pas liées à sa structure secondaire ou tertiare, mais plus précisément à la nature des différents groupes portés par la chaîne polysaccharide et à la combinaison des effets de ces groupes, qui aurait un résultat coopératif multiplicateur de l'activité de chacun d'entre eux vis-à-vis de la thrombine et de l'antithrombine III.

On considère que l'héparine, généralement représentée par la formule II ci-après:

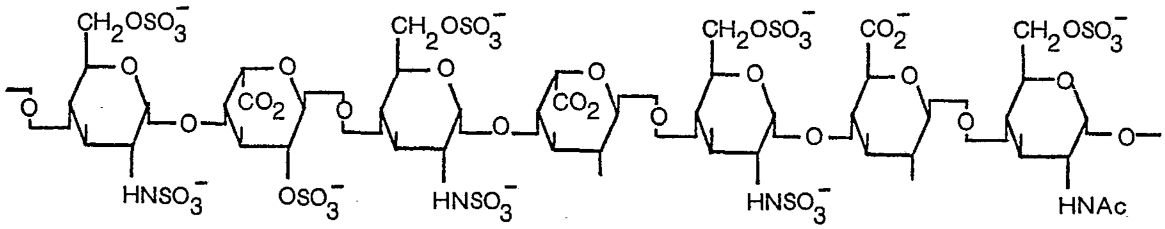

(II)

possède également des motifs non O-sulfatés ou 2-O sulfatés du résidu uronique

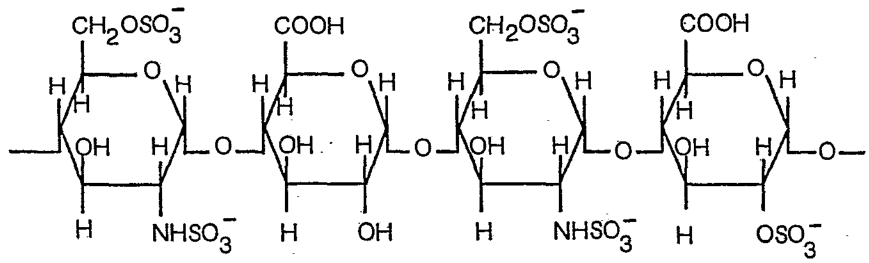

(III)

Par ailleurs, on a préparé des résines échangeuses d'ions utiles pour la chromatographie liquide des mélanges racémiques, lesquelles résines sont essentiellement constituées par des polystyrènes sur lesquels ont été fixés les acides aminés suivants: l'alanine, la valine, la norvaline, la phénylalanine, la leucine, l'isoleucine, la tyrosine, la sérine, la thréonine, l'acide aspartique, la proline, l'hydroxyproline et l'acide glutarrique [cf. en particulier les travaux effectués par VESA et Collab., Zh. Obsch. Khim. SSSR 42 (12) 2780 (1972) et Tr. Akad. Nauk. Lit. SSR. Ser. B. 2—69, 93 (1972) et par PETIT ET JOZEFONVICZ (Jour. Of. Applied Polymer Sci. Vol. 21 2589—2596 (1977)].

Partant de leur hypothèse relative à l'importance de la présence simultanée des groupes figurent dans les formules II et III ci-dessus sur un support macromoléculaire, les Demandeurs ont pu mettre en évidence que de telles résines échangeuses d'ions présentent des applications en tant qu'agents doués d'une action anticoagulante.

Mieux encore, par leur compréhension de ce phénomène et en poussant plus loin leurs études et leurs investigations, des Demandeurs ont pu établir une formule plus générale convenant à tous les polymères non réticulés et comportant dans leur chaîne des groupes substituables. Ils ont pu aboutir ainsi à des produits parfaitement solubles, présentant des propriétés anticoagulantes.

La présente invention a pour objet des produits doués de propriétés anticoagulantes, caractérisés en ce qu'ils sont constitués par des polymères ou copolymères non réticulés et solubles choisis parmi le polystyrène et les polysaccharides, comportant dans leur chaîne des groupes substituables sur lesquels sont fixés de façon statistique, des groupes X et/ou Y et/ou V, où

X désigne le groupe $—SO_3R_1$ ou $—R_3—SO_3R_1$

$R_1$ étant un atome d'hydrogène ou d'un métal physiologiquement compatible,

$R_3$ étant un groupe $—CH_2—CO—NH—R_4—$ dans lequel $R_4$ représente un radical éthylène, arylène ou alkylarylène substitué ou non,

ou $—CH_2$ — 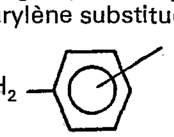 substitué ou non,

Y désigne le groupe $—SO_2—R_2$ ou $—R_3—SO_2—R_2$,

$R_2$ étant le reste d'un acide aminé quelconque dans le cas des polysaccharides et choisi dans le cas du polystyrène dans le groupe qui comprend la méthionine, la cystéine, l'acide cystéique, la

proline, l'hydroxyproline, la sérine, la tyrosine, la benzyloxycarbonyl-lysine, la tertiobutyloxycarbonyl-lysine, l'acide $\varepsilon$-aminocaprïque, la $\beta$-alanine, l'acide $\gamma$-amino-n-butyrique, l'acide $\delta$-amino-n-valérique, substitués ou non, lequel acide aminé est lié au point $—SO_2—$ par sa fonction amine, et

V désigne le groupe $—CH_2—CO—NH—CHR—COOH$,

R étant la chaîne latérale d'un acide aminé,

étant bien entendu que:

a) si X est $—SO_3R_1$, il est obligatoirement accompagné de Y et/ou de V, et
b) V est toujours accompagné de X et/ou de Y.

Conformément à l'invention, dans le cas des polysaccharides, les acides aminés sont choisis parmi ceux qui comportent au moins une fonction carboxylique libre et s'ils possèdant plusieurs fonctions amine, toutes sauf une doivent être bloquées par un groupe électroattracteur physiologiquement acceptable.

Suivant une modalité particulière de ce mode de réalisation, le groupe électroattracteur est avantageusement constitué par le groupe benzyloxycarbonyle ou tertiobutyloxycarbonyle.

Les produits ainsi obtenus qui sont solubles dans l'eau et dans les fluides biologiques permettent de préparer des solutions à action anticoagulante à usage pharmaceutique.

Selon un mode de réalisation avantageux de l'objet de la présente invention, les polymères non réticulés comportant dans leur chaîne des groupes substituables sur lesquels sont fixés les groupes X et/ou Y et/ou V, sont des polystyrènes.

Selon un autre mode de réalisation avantageux de l'objet de l'invention, les polymères non réticulés comportant dans leur chaîne des groupes substituables sur lesquels sont fixés les groupes X et/ou Y et/ou V, sont des polysaccharides.

Suivant une modalité particulière de ce mode de réalisation, les polymères sont constitués par des dextranes. La biocompatibilité des dextranes avec le sang est connue depuis bien longtemps. A. GRONWALL et Collab. [Acta. Physiol. Scand. 7 97 (1944)] ont préconisé leur utilisation comme substitut du plasma sanguin et W. APPEL et Collab. [Angew. Chem. Intern. Ed. *7,* 702 (1968)] ont étudié leur dégradation enzymatique dans le plasma et les tissus sous l'action de la dextranase.

La présente invention a également pour objet un procédé de préparation des produits doués d'une action anticoagulante conformes à la présente invention, lequel procédé, dans le cas où l'on désire obtenir les produits dans lesquels:

$X=—SO_3R_1$

$Y=—SO_2—R_2$

et V est nul

($R_1$ et $R_2$ ayant la même signification que ci-dessus), se caractérise en ce qu'au cours d'une première étape, on prépare un polymère chlorosulfoné $POL—SO_2Cl$ par réaction de l'acide chlorosulfonique sur le polymère dans un solvant approprié, et en ce qu'au cours d'une deuxième étape on transforme les groupes $—SO_2Cl$ en groupes $—SO_3Na$ et en groupes $SO_2AA$ (où AA représente un acide aminé) par réaction avec une quantité appropriée d'un acide aminé en milieu basique.

Suivant une modalité particulière de ce mode de réalisation, dans le cas où on désire obtenir des polymères substitués solubles à action anticoagulante, la sulfonation du polymère non réticulé a lieu dans un milieu organique avantageusement constitué par un solvant chloré et plus particulièrement par le dichlorométhane, le polymère est précipité dans du nitrométhane et la réaction avec l'acide aminé a lieu dans un milieu contenant le mélange eau-dioxane.

Suivant une modalité particulière de ce mode de réalisation, dans le cas de polymères solubles, on élimine les impuretés après la fixation de l'amino-acide par dialyse contre de l'eau et on récupère, si on le désire, le produit fini pur par lyophilisation.

Conformément à l'invention, dans le cas où l'on désire obtenir les produits dans lesquels

$X=—R_3—SO_3R_1$

$V=—CH_2—CO—NH—CHR—COOH$

et Y est nul

($R_1$ et $R_3$ ayant la même signification que ci-dessus), le procédé se caractérise en ce que l'on prépare au cours d'une première étape les dérivés carboxyméthylés des polymères, en ce que l'on fixe au cours de

la deuxième étape les amines appropriées ou le chlorure de benzyle, en ce que l'on fixe les acides aminés au cours d'une troisième étape et en ce que l'on procède à la sulfonation au cours d'une quatrième étape.

Suivant une modalité particulière de ce mode de réalisation, le couplage des amines et/ou des acides aminés sur des dérivés carboxyméthylés des polymères, s'effectue à l'aide de N-éthoxycarbonyl-2-éthoxy 1,2-dihydroquinoléine (EEDQ) ou autre agent de couplage analogue.

Suivant une autre modalité particulière de ce mode de réalisation, l'étape de sulfonation est répétée plusieurs fois si l'on désire augmenter le taux de groupements sulfonates.

Suivant une autre modalité particulière de ce mode de réalisation, le produit fini est purifié par ultrafiltration sous pression.

Conformément à l'invention, dans le cas où l'on désire obtenir les produits dans lesquels:

$X=—R_3—SO_3R_1$

$V=—CH_2—CO—NH—CHR—COOH$

et $Y=—R_3—SO_2—R_2$

R, $R_1$, $R_2$ et $R_3$ ayant la même signification que ci-dessus, le procédé se caractérisé en ce qu'au cours d'une première étape on procéde à la carboxyméthylation, en ce qu'au cours d'une deuxième étape on fixe les amines appropriées ou le chlorure de benzyle, en ce qu'au cours d'une troisième étape on procède à la chlorosulfonation, en ce qu'au cours de la quatrième étape on fixe les acides aminés.

La présente invention a en outre pour objet des compositions pharmaceutiques constituées par, ou contenant les polymères non réticulés substitués par des groupes leur conférant une action anti-coagulante et solubles dans l'eau, lesdits polymères étant présents dans lesdites compositions pharmaceutiques à raison d'une dose thérapeutiquement active.

Outre les dispositions qui précèdent, l'invention comprend encore d'autres dispositions qui ressortiront de la description qui-va suivre.

La présente invention vise plus particulièrement l'utilisation des polymères comportant des groupes X, et/ou Y, et/ou V tels que définis plus haut, fixés sur leur chaîne macromoléculaire, en tant que produits à action analogue à celle de l'héparine.

L'invention pourra être mieux comprise à l'acide du complément de description qui va suivre, qui se réfère à des exemples de préparation et de caractérisation des produits objets de la présente invention, à une étude de l'activité anti-coagulante, et d'autres propriétés analogues à celles de l'héparine de ces produits, et à une évaluation de l'activité propre de chaque type de substituant.

Il doit être bien entendu, toutefois, que les différents exemples, caractéristiques et études qui seront décrits ci-après et représentés sur les dessins annexés, sont donnés uniquement à titre d'illustration de l'objet de l'invention, mais n'en constituent en aucune manière une limitation.

I—Preparation des materiaux et reactifs ayant le polystyrene (PS) comme polymere support

Les matériaux suivants ont été préparés:

a) Produits à action anticoagulante conformes à l'invention

| N° de référence | Nom du produit |
|---|---|
| 1 | PS-hydroxyproline |
| 2 | PS-proline |
| 3 | Ps-alanine |
| 4 | PS-phénylalanine |
| 5 | PS-acide glutamique |
| 6 | PS-méthionine |
| 7 | PS-thréonine |
| 8 | PS-$\alpha$ ou $\varepsilon$-benzyloxycarbonyl-lysine |
| 9 | PS-$\varepsilon$-tertiobutyloxycarbonyl-lysine |
| 16 | PS-acide aspartique |
| 17 | PS-$\beta$ alanine |
| 18 | PS-acide $\varepsilon$-aminocaproïque |

b) Produits d'étude et de comparaison

| N° de référence | Nom du produit |
|---|---|
| 10 | PS-lysine (diamino-acide) |
| 11 | PS—$CH_2$-Proline (l'acide aminé est fixé par l'intermédiaire du pont —$CH_2$—) |
| 12 | PS—$CH_2$-hydroxyproline (l'acide aminé est fixé par l'intermédiaire du pont —$CH_2$—) |
| 13 | PS—$CH_2$-alanine (l'acide aminé est fixé par l'intermédiaire du pont —$CH_2$—) |
| 14 | PS-butylamine (fixation d'une amine et non plus d'un acide aminé) |
| 15 | PS—$SO_3$ (Polystyrène sulfonè) |

Le polystyrène de départ est un copolymère du styrène et de 2% de divinylbenzène utilisé sous forme de billes de 200 à 400 mesh de diameter, soit entre 0,037 et 0,074 mm de diamètre.

Les acides aminés utilisés sont des réactifs "FLUKA Puriss".

c) Préparation de produits, à action anticoagulante, solubles 1ère étape: synthèse du polystyrène chlorosulfoné

On dissout 25 g de polystyrène non réticulé dans 200 ml de dichlorométhane. On ajoute alors sous forte agitation, 16 ml d'acide chlorosulfonique et on laisse la réaction se poursuivre pendant 7 heures à 40°C. On ajoute alors du nitrométhane pour précipiter tout le polymère. On filtre et on lave au nitrométhane pour éliminer toute trace d'acide chlorosulfonique. Le polymère est alors séché sous vide. Le taux des groupes chlorosulfonyle est déterminé de la façon suivante: 200 mg de polystyrène chlorosulfoné sont hydrolysés avec 50 ml d'une solution 1 M de NaOH pendant 24 heures au reflux. Après acidification, les ions $Cl^-$ sont titrés par une solution 0,1 M de $AgNO_3$ en utilisant une électrode indicatrice d'argent.

2ème étape: fixation de l'amino-acide

50 mmoles d'amino-acide (sous forme de sel de sodium) sont dissoutes dans 100 ml du mélange 3-2 eau-dioxane. Le pH est mesuré et on ajoute 10 g du polymère précédent. Le pH est ensuite

maintenu à sa valeur initiale telle que mesurée, par addition de soude 2 M. On arrête la réaction quand le pH reste stable. Le milieu réactionnel est alors dialysé contre de l'eau et on récupère le produit pur par lyophilisation.

II—Preparation de materiaux ayant le dextrane comme polymere support

Synthèse des composés non réticulés, solubles

a) Préparation de:

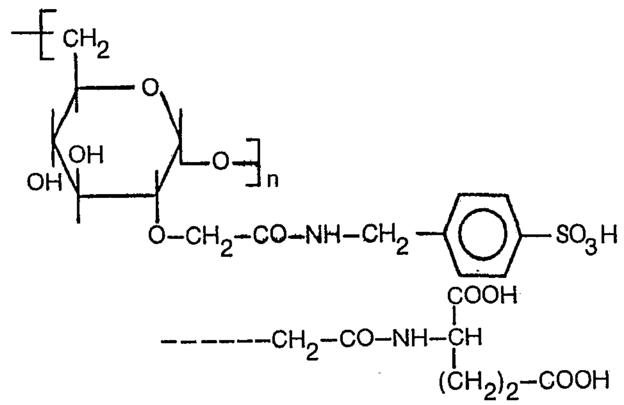

1ère étape: Carboxyméthylation des dextranes

(On opère en s'inspirant des travaux de E. ANTONINI et Collab. [Giorn. Biochi. *14* 88 (1965)]).

Dans un ballon de 1 l à fond plat muni d'un système d'agitation, immergé aux 2/3 dans un bain d'huile, et surmonté d'un réfrigérant, on dissout à la température ambiante 16,2 g de dextrane (0,1 mole) dans 500 ml de soude 12 M (6 moles). On a porte à une température voisine de 70°C et on maintient à cette température pendant 3 heures. On introduit alors peu à peu dans ce mélange toujours maintenu à environ 70°C, 283,5 g de $ClCH_2COOH$ (3 moles); durée d'introduction=une demi-heure environ). On laisse sous agitation (toujours à 70°C environ) pendant 24 heures. On laisse alors revenir à la température ambiante, puis on précipite dans 1,5 litre de méthanol, on sépare ensuite le produit précipité par filtration, on le lave avec un peu de méthanol, puis on le redissout dans environ 100 ml d'eau distillée. On précipite à nouveau dans environ 500 ml de méthanol, on filtre, lave et l'on sèche dans une étuve sous vide à 40°C environ.

2ème étape: Fixation de la benzylamine

Dans un ballon à fond plat de 200 ml, muni d'un système d'agitation, on dissout, à la température ambiante, 5 g de carboxyméthyl-dextrane (préparé au cours de la première étape) dans 20 ml d'eau distillée. On ajuste le pH du milieu à environ 4 avec un peu d'acide chlorohydrique concentré. On verse alors lentement (durée de l'addition: 15 minutes environ) 6 g d'EEDQ dissous dans 50 ml d'ethanol absolu. On maintient l'agitation pendant une demi-heure puis l'on ajoute 3 ml de benzylamine et on laisse sous agitation une nuit. On verse alors le mélange dans 700 ml d'acétone, on filtre le précipité formé, on le lave à l'acétone et l'on sèche à l'étuve sous vide.

3ème étape: Fixation de l'acide glutamique

On dissout 2 g du produit préparé au cours de la deuxième étape dans 10 ml d'eau distillée et on ajuste le pH à 4 par quelques gouttes d'HCl concentré. On y ajoute alors lentement 1,7 g d'EEDQ dissous dans 13 ml d'acétone. On laisse sous agitation pendant une demi-heure puis on ajoute dans ce mélange réactionnel 10 ml d'une solution aqueuse contenant 1,6 g d'acide glutamique et 0,87 g de soude. On laisse sous agitation pendant une nuit à la température ambiante. Le mélange est alors acidifié (pH compris entre 4 et 5) à l'aide d'HCl, puis on précipite dans 800 ml de méthanol. Le produit précipité est séparé par filtration, lavé au méthanol, puis séché à l'étuve sous vide pendant une nuit.

4ème étape: Sulfonation

On pèse 1,2 g du produit obtenu au cours de la troisième étape et on le met -sous agitation- dans 100 ml de nitrométhane. On ajoute alors lentement 0,4 d'acide chlorosulfonique et on laisse sous agitation pendant 2 heures. On sépare le produit formé par filtration, on lave au nitrométhane, on met le produit pendant 2 heures dans de la soude M, on le précipite au méthanol, on lave et on sèche sous vide.

5ème étape: Purification

Le produit sec obtenu au cours de la quatrième étape est traité dans une cellule d'ultrafiltration (membrane 5000) en présence d'eau bidistillée, sous une pression de 2 bars, pendant 3 jours.

b) Préparation de:

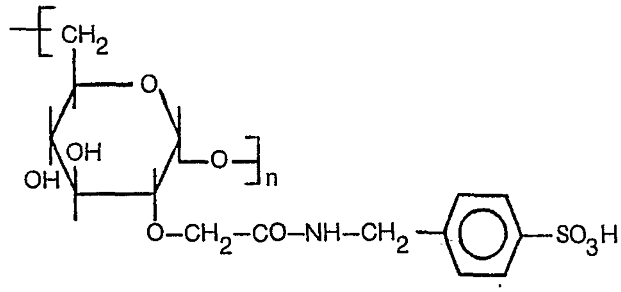

On effectue dans l'ordre les étapes 1), 2), 4) et 5) de l'Exemple III a).

III—Tests de coagulation

Le plasma pauvre en plaquettes (PPP) est préparé à partir de plasma humain frais par centrifugation (4°C—10 000 g—30 minutes). Il est stocké à −20°C, dégelé par petites quantités et maintenu alors à 4°C.

Le fibrinogène utilisé est du fibrinogène bovin purifié (Behring) à une concentration de 6 g/l dans NaCl à 0,85%. La solution est faite juste avant l'emploi et conservée à 37°C.

La thrombine (Roche, 50 NIH/mg), l'antagoniste de l'héparine [polybrène ou polylysine (Sigma)] sont dilués extemporanément dans du tampon de MICHAELIS et conservés à 4°C (pour la thrombine) ou à la température ambiante (pour le polybrène et la polylysine). La reptilase (Stago) est diluée dans l'eau distillée et conservée à 37°C. L'antithrombine III (Kabi) est en solution dans l'eau distillée et conservée à 4°C.

Les polymères sont en solution dans du tampon de Michaelis additionné de 1 g/litre d'un agent émulsifiant (Lensex TA 01—NP 40 Shell).

Tous les temps de coagulation sont déterminés à 37°C en tube de verre par observation directe.

IV—Etude de l'activite anticoagulante

L'activité anticoagulante des produits couforming à l'invention a été étudiée par détermination des temps de thrombine de plasma décalafié et pauve en plaquettes (PPP) ou de solution de fibronogène en présence de la solution à étudier en concentration variable. Des études préliminaires de plusieurs couposs PS—$SO_3$ contenant des proportions variées de groupes —$SO_3Na$, ont montré que le temps de thrombine ne dépend que de la quantité de groupes —$SO_3Na$ présents dans un volume donné de PPP au moment de test. En conséquence, pour pouvoir comparer directement l'activité anticoagulante de différentes solutions, les temps de thrombine ont été portés en fonction de la quantité de produit exprimée en concentration de —$SO_3Na$.

Les résultats sont représentés sur la figure 1. On a porté en ordonnée le temps de thrombine en secondes, et en abscisse la concentration de la solution de polymère (exprimée en nombre de groupes $SO_3^-$/ml) pour 30 U/ml de thrombine (figure 1a), 50 U/ml de thrombine (figure 1b) et 75 U/ml de thrombine (figure 1c).

Les courbes 1 correspondent au produit PS—$SO_3$ avec du PPP.

Les courbes 2 correspondent au produit PS—$SO_2$-Glu avec du PPP.

Les courbes 3 correspondent au produit PS—$SO_3$ avec la solution de fibronogène.

Les courbes 4 correspondent au produit PS—$SO_2$-Glu avec la solution de fibrinogène.

Une solution de 0,1 ml de polymère (de concentration variable) est incubée avec 0,2 ml de PPP (courbes continues sur la figure 1) ou d'une solution de fibronogène (courbes en traits discontinus sur la figure 1) pendant 30 minutes à 37°C. On ajoute alors 0,1 ml de thrombine et on mesure le temps de coagulation.

En examinant la figure 1, on constate immédiatement que l'effet anticoagulant du produit PS—$SO_2$-Glu est plus grand que celui du produit PS—$SO_3$.

Dans tous les cas, les contrôles supplémentaires suivants ont été faits:

Determinatiou du temps de coagulation par la reptilase, de PPP ou d'une solution de fibrinogène incubés avec le polymère. Aucune différence significative n'a pu être mise en évidence entre les temps de reptilase obtenus en présence ou en l'absence de polymère, ainsi que cela ressort des résultats représentés sur la figure 2. On a tracé sur cette figure 2 le temps de coagulation en secondes en fonction de la concentration de la solution de polymère (exprimée en nombre de groupes de $SO_3^-$/ml). La solution de polymère (0,1 ml) est incubée avec 0,2 ml de PPP pendant 30 minutes à 37°C. On ajoute alors 0,1 ml de thrombine à 9 U/ml (courbe continue) ou de reptilase (courbe en traits discontinus). La concentration de la thrombine a été choisie pour donner un temps témoin identique à la reptilase, à savoir 20 secondes. Les courbes 1 correspondent au PS—$SO_3$ et les courbes 2 à un polymère conforme à l'invention (PS—$SO_2$-Glu).

Les courbes obtenues avec le plasma, pour différentes concentrations de thrombine ont

également été transposées en quantité de thrombine inactivée en fonction de la quantité de produit présente dans le test. En effet, en première approximation, on peut considérer qu'un temps de thrombine allongé traduit l'inactivation d'une fraction de la thrombine initiale et déterminer cette fraction en comparant le temps de coagulation obtenu avec une courbe de temps témoins mesurés avec des quantités de thrombine variables. La figure 3 montre les courbes ainsi obtenues.

Les courbes 1 correspondent au polymère PS—$SO_3$.

Les courbes 2 correspondent au polymère conforme à l'invention PS—$SO_2$-Glu.

On a porté en ordonnée la thrombine inactivée en U/ml et en abscisse la concentration de la solution de polymère exprimée en nombre de groupes $SO_3^-$/ml. La quantité de thrombine inactivée a été évaluée à partir d'une courbe d'étalonnage de temps témoins (0,2 ml de PPP sont incubés avec 0,1 ml de tampon de MICHAELIS à 37°C. On ajoute 0,1 ml de thrombine—de concentration variable—et on mesure le temps de coagulation). Les temps de thrombine, mesurés après incubation de PPP avec les solutions de polymère, sont reportés sur cette courbe d'étalonnage, ce qui permet d'évaluer la concentration d'enzyme active, donc la quantité correspondante de thrombine inactivée.

Les courbes représentées sur les figures 3a, 3b, 3c, montrent clairement que la proportion de thrombine inactivée augmente avec la quantité de polymère présente dans le test.

La comparaison des temps de thrombine obtenus dans les mêmes conditions pour le PPP et la solution de fibrinogène (cf. figures 1 et 3) montre que l'effet anticoagulant du polymère ne se manifeste qu'en présence d'un facteur plasmatique absent de la solution de fibrinogène.

L'ensemble de ces résultats suggère que l'activité anticoagulante de ces polymères est une activité antithrombique impliquant l'antithrombine III comme cofacteur plasmatique. En d'autre termes, ces polymères sont des matériaux anticoagulants "héparine libre".

V—Etablissement de la loi generale de variation entre la quantite de thrombine inactivee et la quantite de produit PS—$SO_2$AA presente

L'activité anticoagulante des différents échantillons a été étudiée par détermination des temps de thrombine de PPP incubé avec des quantités croissantes de polymère conforme à l'invention. Au préalable, pour vérifier que l'activité anticoagulante de chaque polymère est analogue à celle des échantillons décrits dans le paragraphe IV ci-dessus, et dans le paragraphe IX ci-après, les temps de reptilase de PPP incubé avec le polymère et les temps de thrombine de la solution de fibrinogène incubée avec le polymère ont été déterminés. Ils sont tous sensiblement égaux aux temps témoins correspondants (déterminés en l'absence de polymère).

L'allongement du temps de thrombine correspondant à l'inactivation d'une fraction de la thrombine initiale, la comparaison entre une courbe d'étalonnage et le temps de coagulation déterminé en présence d'une certaine quantité de produit, permet de déterminer la quantité de thrombine inactivée par cette quantité de produit présente dans le PPP. La figure 4 montre la variation de la quantité de thrombine inactivée en fonction de la concentration de la solution de polymère pour une série de composés contenant de la proline en proportion variable par rapport aux groupes sulfonate. La concentration de la solution de polymere est ici exprimée en milliéquivalents de groupes —$SO_3$Na par millilitre de solution, ce qui permet de comparer directement l'effet des groupes proline et montre que l'activité anticoagulante augmente avec le taux de ces groupes.

La figure 4 représente en effet la quantité de thrombine inactivée en fonction de la concentration de la solution de polymère PS—$SO_2$-proline (exprimée en meq-$SO_3$Na/ml)

pour 2,59 meq de proline par g de polymère: (courbe 1)
pour 2,09 meq de proline par g de polymère: (courbe 2)
pour 1,62 meq de proline par g de polymère: (courbe 3)
pour 0,93 meq de proline par g de polymère: (courbe 4)

Une même série de courbes a été obtenue pour chaque acide aminé.

La figure 5 montre la variation de la quantité de thrombine inactivée en ordonnée, en fonction de la concentration de polymère (en mg/ml) pour un polymère portant des groupes acide glutamique et pour quatre concentrations de thrombine:

—12 U/ml (courbe 1)
—30 U/ml (courbe 2)
—50 U/ml (courbe 3)
—75 U/ml (courbe 4)

Les mêmes courbes ont été établies pour tous les autres produits conformes à l'invention, préparés, et on a constaté que la loi de variation est dans tous les cas la même. Cette variation est tout d'abord linéaire, ce qui montre que la quantité de thrombine inactivée est proportionnelle à la quantité de polymère présent quelle que soit la concentration de thrombine utilisée. Cependant, pour atteindre 100% d'inactivation, un excès de polymère est toujours nécessaire. L'extrapolation de la tangente à l'origine permet pour chaque polymère, de déterminer la quantité de produit théoriquement nécessaire

pour inactiver totalement une certaine quantité de thrombine. Ceci résulte clairement de la figure 6 où les grandeurs ont été portées en coordonnées réduites: en ordonnée, la thrombine inactivée en % du total, et en abscisse la quantité de polymère en % de la quantité théoriquement nécessaire pour inactiver toute la thrombine présente.

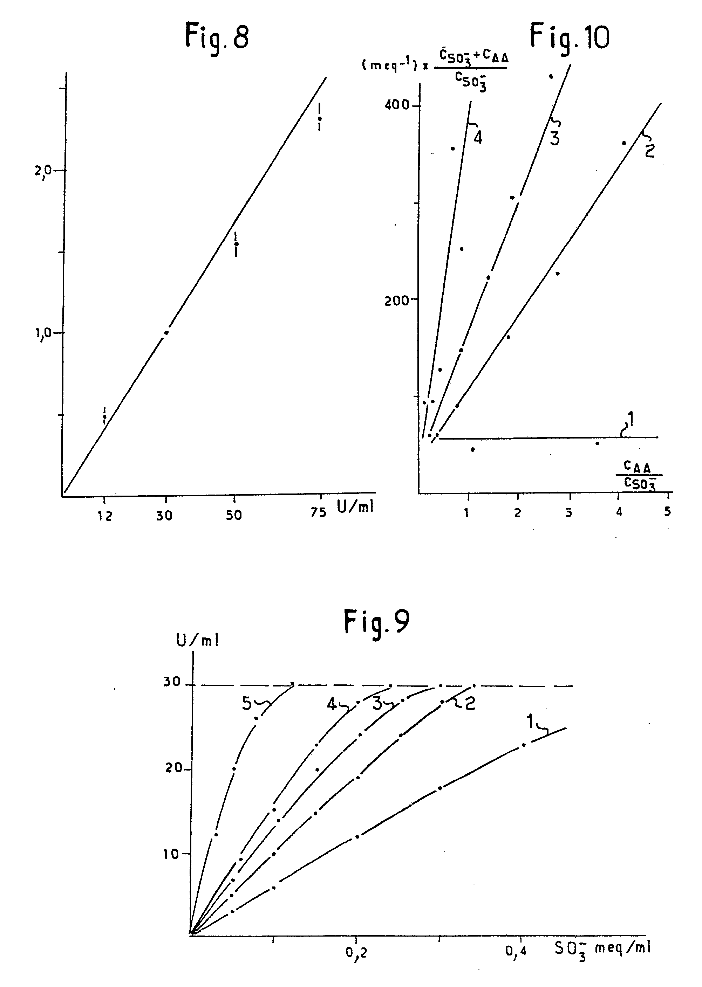

La figure 7 permet de vérifier que cette loi de proportionnalité est générale. En effet, chaque point correspond ici à la quantité de polymère nécessaire pour inactiver 30 unités de thrombine en fonction de la quantité du même polymère nécessaire pour inactiver 12 unités de thrombine. Tous ces points sont sensiblement alignés et très voisins de la droite de pente 30/12 correspondant à la proportionnalité, ce qui montre que la loi de variation est valable pour tous les amino-acides étudiés. Il est alors possible de déterminer une activité pour chaque produit comme l'inverse de la quantité de produit nécessaire pour inactiver 30 unités de thrombine. En utilisant cette valeur comme référence, on peut alors montrer que la loi de proportionnalité est valable pour des concentrations de thrombine variant de 12 à 75 unités/ml, comme le montre la figure 8 où on a porté en abscisse la concentration de la thrombine et en ordonnée le rapport:

$$\frac{\text{Polymère nécessaire pour inactiver X unités de thrombine}}{\text{Polymère nécessaire pour inactiver 30 unités de thrombine}}$$

En résumé, il résulte clairement des figures 7 et 8:

—que l'on arrive à inactiver toute la thrombine présente à condition d'ajouter une quantité suffisante de polymère conforme à l'invention

—que la même loi d'inactivation est suivie par tous les produits conformes à l'invention, quel que soit l'aminoacide (AA) et quelle que soit la quantité de cet AA présente dans le produit

VI—Cas des diamino-acides (lysine)

On a représente sur la figure 9, les courbes de variation de la quantité de thrombine inactivée (en ordonnée en U/ml) en fonction de la quantité de produit présent dans le test.

La courbe 1 concerne le polymère PS—$SO_2$-Lysine
La courbe 2 concerne le polymère sulfoné PS—$SO_3$
La courbe 3 concerne le polymère PS—$SO_2$-Proline
La courbe 4 concerne le polymère PS—$SO_2$-$\alpha$ Z Lysine
La courbe 5 concerne le polymère PS—$SO_2$-Glutamique
Z étant le groupe électroattracteur benzyloxycarbonyle.

La quantité de résine étant exprimée en concentration de groupes sulfonate par millilitre de solution pour tout les produits, les courbes supérieures à celle d'un polymère ne contenant que des groupes sulfonate (courbe 2) indiquent que l'amino-acide correspondant confère au polymère sur laquelle il est fixé, une activité anticoagulante supplémentaire par rapport à celle de ses seuls groupes sulfonate. C'est le cas de tous les amino-acides sauf la lysine. Dans ce cas, au contraire, la courbe est inférieure à celle d'un produit ne contenant que des groupes sulfonates (PS—$SO_3$), c'est-à-dire que la présence de la lysine sur le polymère revient à "neutraliser" une partie de l'activité anticoagulante des sites sulfonate. Ceci est à rapprocher de l'effet inhibiteur, décrit au paragraphe IX ci-après, observé avec la polylysine dont les sites amino- des chaînes latérales, protonés à pH 7,35, neutralisent les sites négatifs, notamment sulfonates, d'un polymère PS—$SO_3$, ou PS—$SO_2$AA, ou de l'héparine.

Par contre, quand on a bloqué une fonction amine de la lysine (courbe 4), cette neutralisation n'est plus possible et le polymère retrouvé pleinement ses propriétés.

VII—Cas des polymères PS—$CH_2$—AA (L'acide amine est lie aux chaines macromoleculaires par l'intermediaire du pont —$CH_2$—)

Cette étude a porté sur trois polymères contenant respectivement de la proline, de l'hydroxyproline et de l'alanine (cf. Tableau I: composés 11, 12 et 13). Dans chaque cas, les temps de thrombine de PPP incubé avec des quantités variables de polymère ont été déterminés. Ils ont toujours été trouvés sensiblement égaux au temps témoin, qu'elle que soit la quantité de polymère présent.

VIII—Evaluation de l'activite propre de chaque type de substituant

A partir de l'activité de chaque polymère définie précédemment par rapport à 30 unités de thrombine, on peut déduire l'activité rapportée à l'unité de thrombine (cf. Tableau II). Si les effets des différents substituants du polymère sont additifs, l'activité "a" peut s'exprimer par la relation (1) ci-après:

$$a(C_{SO_3}+C_{AA})=(a_{SO_3^-}XC_{SO_3^-})+(a_{AA}\times C_{AA}) \qquad (1)$$

où $a_{SO_3^-}$ et $a_{AA}$ sont les activités respectivement d'un groupe sulfonate et d'un groupe —$SO_2$-amino-acide, $C_{SO_3^-}$ et $C_{AA}$ étant leurs concentrations respectives.

La figure 10 montre les droites obtenues en portant l'activité du polymère "a", multipliée par le rapport du nombre total de sites et du nombre de sites sulfonates

$$\frac{C_{SO_3^-}+C_{AA}}{C_{SO_3^-}} \quad \text{(en ordonnée)}$$

en fonction du rapport des nombres de sites —$SO_2$-aminoacides et sulfonate

$$\frac{C_{AA}}{C_{SO_3^-}} \quad \text{(en abscisse)}$$

Ces droites ont une ordonnée à l'origine commune égale à l'activité d'un groupe sulfonate ($a_{SO_3^-}$) et des pentes égales aux activités des groupes amino-acides ($a_{AA}$).

La droite 1 correspond au polymère PS—$SO_2$-But-$NH_2$

La droite 2 correspond aux polymères PS—$SO_2$-alanine
PS—$SO_2$-phènylalanine

La droite 3 correspond aux polymères PS—$SO_2$-Z Lysine
PS—$SO_2$-hydroxyproline
PS—$SO_2$-méthionine
PS—$SO_2$-proline
PS—$SO_2$-thréonine

La droite 4 correspond au polymère PS—$SO_2$-Acide glutamique.

Les activités des différents types de substituants sont regroupées dans le Tableau II et le Tableau III ci-après:

A—Polymère support: Polystyrène

TABLEAU II

| Substituant | Coefficient d'activité en $(meq)^{-1}$ $uTh^{-1}$ | Substituant | Coefficient |
|---|---|---|---|
| —$SO_2$ ASP<br>—$SO_2$ Glu | 550<br>370 | $SO_2$Z Lys | 130 |
| —$SO_2$ ε-aminocaproïque<br>—$SO_2$ β-alanine | 200 à 300 | —$SO_2$—Ala<br>—$SO_2$-Phe | compris entre 60—80 |
| —$SO_2$OH Prol<br>—$SO_2$Prol<br>—$SO_2$Met<br>—$SO_2$Thr | compris entre 120 et 150 | —$SO_3$' | 60 |
| | | $SO_2$But $NH_2$ | 0 |

On voit clairement que:

—l'activité de la butylamine est nulle, ce qui semble indiquer que le groupe sulfamide seul n'est pas suffisant pour conférer au produit une activité antithrombique et que les substituants dépourvus de fonction acide carboxylique n'ont aucun effet sur cette activité.

—Les groupes alanine et phénylalanine ont une activité comprise entre 60 et 80 $meq^{-1}$, voisine de celle de groupe sulfonate, 60 $meq^{-1}$, ce qui pourrait indiquer qu'une chaîne latérale hydrocarbonée n'a pratiquement aucun rôle et que l'activité de la fonction carboxylique de l'amino-acide est du même ordre de grandeur que celle du groupe sulfonate. La faible différence entre l'alanine et la phénylalanine s'explique probablement par le caractère hydrophobe plus marqué de la chaîne latérale de la phénylalanine par rapport à l'alanine.

—La lysine dont la fonction amine de la chaîne latérale est substituée par un groupe attracteur d'électrons (groupe Z) a une activité positive, mais difficile à définir avec précision dans la mesure où il n'a pas été possible de synthétiser des polymères contenant plus de groupes amino-acide que de groupes sulfonate. Le même problème s'est posé pour la lysine, mais dans ce cas, l'activité, très mal définie également, est négative.

—Les amino-acides dont la chaîne latérale contient un hétéroatome, comme l'hydroxyproline, la thréonine et la méthionine, ont une activité plus élevée comprise entre 120 et 150 $meq^{-1}$.
La proline semble également pouvoir être rattachée à ce groupe. Il faut noter que deux de ces amino-acides, proline et hydroxyproline, fixés sur le polystyrène par un groupe méthylène polymère PS—$CH_2$AA) ne confèrent aucune activité anticoagulante.
—Les acides dicarboxyliques, comme l'acide glutamique ou l'acide aspartique, ont une activité plus élevée que tous les autres $\alpha$-amino-acides, probablement liée à leurs deux fonctions carboxyliques.
—Les acides aminés dans lesquels la fonction amine et la fonction carboxylique sont séparées par plusieurs groupes méthylène, comme par exemple la $\beta$-alanine, ou l'acide $\varepsilon$-aminocaproïque, ont une activité d'autant plus élevée que le nombre de groupes méthylène est plus grand, probablement liée à un effet de "bras" de la chaîne carbonée rendant plus accessible la fonction carboxylique.

L'ensemble de ces résultats permet de tirer un certain nombre de conclusions. Le groupe sulfamide n'est pas suffisant pour qu'il y ait une activité anticoagulante; cependant, il semble qu'il soit nécessaire puisque les polymères PS—$CH_2$—AA n'ont aucune activité.

Les groupes sulfonates, en revanche, sont suffisants pour qu'il y ait activité anticoagulante (cf. également le travail de GREGOR précédemment cité), mais celle-ci est faible à très faible, au maximum 15% de celle de l'héparine, quand tous les sites du polymère (PS) sont substitués par des groupes $SO_3^-$.

Par contre, quand la chaîne macromoléculaire porte à la fois des groupes sulfonates, des amino-acides fixés par un pont sulfamide et/ou amide et que les amino-acides ont une chaîne latérale favorable, l'activité anticoagulante du produit peut être très importante.

B—Polymère support: Dextrane

TABLEAU III

Etude du temps de coagulation en fonction de la nature de $R_3$ et de la quantité de $SO_3^-$ dans le polymère

| | Nature de $R_3$ | Meq de $SO_3^-$ dans la solution initiale de polymère nécessaire pour avoir le temps de thrombine de 20 secondes (concentration de thrombine 30 U/ml) |
|---|---|---|
| 1 | —$CH_2$ 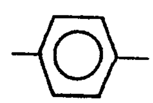 | $17.10^{-3}$ |
| 2 | —$CH_2$CO—NH—$CH_2$ 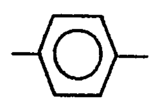 | $2,5.10^{-3}$ |
| 3 | $-CH_2-C(=O)-NH-CH_2-$ | $200.10^{-3}$ |

Témoin sans polymère: 7 secondes.

Il résulte de la comparaison de ces trois produits que c'est le produit n° 2 qui s'est avéré le meilleur: temps de 20 secondes pour seulement une concentration de $2,5.10^{-3}$ en meq de $SO_3^-$.

Pour des produits conforms à l'invention on a trouvé ainsi, à partir de la mesure des temps de thrombine, les coefficients d'activité suivants:

| Substituant | Coefficient d'activité |
|---|---|
| —$CH_2$CO—NH—$CH_2$— 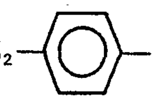 $SO_3^-$ | $12.10^3$ $meq^{-1}$ u $Th^{-1}$ |
| —$CH_2$CO-Thréonine | $36.10^3$ $meq^{-1}$ u $Th^{-1}$ |

IX—Autres proprietes analogues a l'heparine des composes conformes a l'invention

A—Etude de l'action des inhibiteurs

Les inhibiteurs de l'héparine (polybrène et polylysine) peuvent inhiber totalement l'effet anticoagulant des polymères conformes à l'invention.

Les résultats sont représentés sur les fig. 11a et 11b annexée (dans laquelle on a porté en ordonnée le temps de thrombine en secondes, et en abscisse la concentration de l'inhibiteur en mg/ml). Le système expérimental est le suivant: la solution de polymère (0,1 ml) est incubée avec 0,2 ml de PPP et 0,1 ml d'une solution d'inhibiteur (de concentrations variables) pendant 30 minutes à 37°C. On ajoute alors 0,1 ml de thrombine (12 U/ml) et on mesure le temps de coagulation. Les courbes 1 concernent le polybrène et les courbes 2 la polylysine.

La figure 11a représente les résultats obtenus avec le PS—$SO_2$-Glu et la figure 11b ceux obtenus avec le polymère PS—$SO_3$. On voit sur ces figures que cette inhibition n'est totale qu'à condition qu'il y ait un excès de charge positives de l'inhibiteur par rapport aux charges négatives du polymère, alors que pour l'héparine elle est totale sans excès. Il est à noter également qu'il faut un excès plus grand avec le polybrène qu'avec la polylysine. Ces résultats indiquent qu'une grande proportion des sites négatifs accessibles du polymère sont impliqués dans son activité anticoagulante.

B—Etude de l'inactivation de la thrombine par les polymères en présence ou en l'absence d'antithrombine III

Cette seconde étude a été faite en deux séries:

a) les polymères sont incubés avec de la thrombine ou successivement avec de la thrombine et du polybrène.

b) Les polymères sont incubés avec de la thrombine et de l'antithrombine III ou successivement avec de la thrombine, de l'antithrombine III et du polybrène. Dans tous les cas, le mélange est alors centrifugé et le surnageant est ajouté à du PPP ou à une solution de fibrinogène. On mesure alors le temps de coagulation (cf. Tableau IV ci-après). Les résultats obtenus montrent qu'en l'absence d'antithrombine III, la thrombine est inactivée de façon réversible par le polymère; son activité réapparaît en présence de polybrène. En revanche, en présence d'antithrombine III, l'inactivation de la thrombine par le polymère est irréversible, comme c'est le cas avec l'héparine.

TABLEAU IV

Etude de l'inactivation de la thrombine par les polymeres en presence ou en l'absence d'antithrombine III

| Echantillon | | Thrombine + tampon (a) * | Thrombine + polybrene (b) * | Thrombine + antithrombine + tampon (c) * | Thrombine + antithrombine + polybrène (d) * |
|---|---|---|---|---|---|
| PS—$SO_3$ | PPP | 70 | 45 | 250 | 64 |
| | Solution de fibrinogène | 110 | 44 | 300 | 60 |
| PS—$SO_2$Glu | PPP | 112 | 45 | 300 | 65 |
| | Solution de fibrinogène | 150 | 47 | 360 | 62 |

* temps de coagulation en secondes

Système expérimental: une solution (0,2 ml) de polymère dans du tampon est incubée à 0°C avec 0,2 ml de thrombine à 5 unités/ml (a) (b) ou avec 0,016 ml d'antithrombine III à 25 unités/ml et 0,2 ml de thrombine à 5 unités/ml (c) (d). Après 5 minutes, 0,2 ml de tampon (a) (c) ou 0,2 ml d'une solution de polybrène (40 mg/ml dans du tampon) (b) (d) sont ajoutés. Après 5 minutes à 0°C, la solution est centrifugée et 0,3 ml de surnageant sont ajoutés à 0,2 ml de PPP ou 0,2 ml de solution de fibrinogène à 37°C. Le temps de coagulation (en sec.) est alors mesuré. Temps témoin: 45 secondes en l'absence d'antithrombine III et 60 secondes en présence d'antithrombine III.

Il résulte de la description qui précède que les produits conformes à la présente invention présentent une activité anticoagulante très marquée permettant d'detenir des produits à action anticoagulante, solubles, à usage pharmaceutique proprement dit.

**Revendications**

1. Produits doués de propriétés anticoagulantes, caractérisés en ce qu'ils sont constitués par des polymères, homopolymères ou copolymères non réticulés et solubles choisis parmi le polystyrène et les

polysaccharides, comportant dans leur chaîne des groupes substituables sur lesquels sont fixés de façon statistique des groupes X et Y et/ou V, où

X désigne le groupe $—SO_3R_1$ ou $—R_3—SO_3R_1$
$R_1$ étant un atome d'hydrogène ou d'un métal physiologiquement compatible,
$R_3$ étant un groupe $—CH_2—CO—NH—R_4—$ dans lequel $R_4$ représente un radical éthylène, arylène ou alkylarylène substitué ou non,

ou $—CH_2$ — 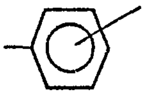 substitué ou non,

Y désigne le groupe $—SO_2—R_2$ ou $—R_3—SO_2—R_2$,
$R_2$ étant le reste d'un acide aminé quelconque dans le cas des polysaccharides et choisi dans le cas du polystyrène dans le groupe qui comprend la méthionine, la cystéine, l'acide cystéique, la proline, l'hydroxyproline, la sérine, la tyrosine, la benzyloxycarbonyl-lysine, la tertiobutyloxy-carbonyl-lysine, l'acide $\varepsilon$-aminocaproïque, la $\beta$-alanine, l'acide $\gamma$-amino-n-butyrique, l'acide $\delta$-amino-n-valérique, substitués ou non, lequel acide aminé est lié au pont $—SO_2—$ par sa fonction amine, et
V désigne le groupe $—CH_2—CO—NH—CHR—COOH$,
R étant la chaîne latérale d'un acide aminé,

étant bien entendu que:

a) si X est $—SO_3R_1$, il est obligatoirement accompagné de Y et/ou de V, et
b) V est toujours accompagné de X et/ou de Y.

2. Produits selon la Revendication 1, caractérisés en ce que, dans le cas des polysaccharides, les acides aminés sont choisis parmi ceux qui comportent au moins une fonction carboxylique libre.

3. Produits selon la Revendication 1, caractérisés en ce que l'acide aminé est un acide aminé dicarboxylique.

4. Produits selon l'une quelconque des Revendications 1 à 2, caractérisés en ce que lorsque les acides aminés possédent plusieurs fonctions amines, toutes sauf une doivent être bloquées par un groupe électroattracteur physiologiquement acceptable.

5. Produits selon la Revendication 4, caractérisés en ce que le groupe électroattracteur est avantageusement constitué par un groupe benzyloxycarbonyle ou tertiobutyloxycarbonyle.

6. Produits selon la Revendication 1, caractérisés en ce que les polymères sont des dextranes.

7. Procédé de préparation des produits selon l'une quelconque des Revendications 1 à 6, caractérisé—lorsqu'on désire obtenir les produits dans lesquels

$X=—SO_3R_1$

$Y=—SO_2—R_2$

et V=nul

($R_1$ et $R_2$ ayant la même signification que précédemment)—en ce qu'au cours d'une première étape, on prépare un polymère chlorosulfoné $POL—SO_2Cl$ par réaction de l'acide chlorosulfonique sur le polymère, dans un solvant approprié, et en ce qu'au cours d'une deuxième étape, on transforme les groupes $—SO_2Cl$ en groupes $—SO_3Na$ et en groupes $—SO_2AA$ (où AA représente un acide aminé) par réaction avec une quantité appropriée d'un acide aminé en milieu basique.

8. Procédé selon la Revendication 7, caractérisé en ce que la sulfonation du polymère non réticulé a lieu dans un milieu organique avantageusement constitué par un solvant chloré, le polymère est précipité dans du nitrométhane et la réaction avec l'acide aminé a lieu dans un milieu contenant le mélange eau-dioxane.

9. Procédé selon la Revendication 3, caractérisé en ce que le milieu de sulfonation est constitué par du dichlorométhane.

10. Procédé selon la Revendication 8, caractérisé en ce qu'on élimine les impuretés après la fixation de l'amino-acide, par dialyse contre de l'eau et on récupère, si on le désire, le produit fini pur par lyophilisation.

11. Procédé de préparation de produits selon l'une quelconque des Revendications 1 à 6, caractérisé lorsqu'on désire obtenir les produits dans lesquels:

$X = -R_3-SO_3R_1$

$V = -CH_2-CO-NH-CHR-COOH$

et Y est nul

(R, $R_1$ et $R_3$ ayant la même signification que précédemment), en ce que l'on prépare, au cours d'une première étape, les dérivés carboxyméthylés des polymères, en ce que l'on fixe, au cours de la deuxième étape, les amines appropriées ou le chlorure de benzyle, en ce que l'on fixe les acides aminés au cours de la troisième étape et en ce que l'on procède à la sulfonation au cours de la quatrième étape.

12. Procédé selon la Revendication 11, caractérisé en ce que le couplage des amines et/ou des acides aminés sur des dérivés carboxyméthylés de polymères s'effectue à l'aide de N-éthoxycarbonyl-2-éthoxy 1,2-dihydroquinoléine (EEDQ).

13. Procédé selon la Revendication 11, caractérisé en ce que l'étape de sulfonation est répétée plusieurs fois si l'on désire augmenter le taux de groupements sulfonates.

14. Procédé selon la Revendication 11, caractérisé en ce que le produit fini est purifié par ultrafiltration sous pression.

15. Procédé de préparation des produits selon l'une quelconque des Revendications 1 à 6, caractérisé, lorsqu'on désire obtenir les produits dans lesquels:

$X = -R_3-SO_3R_1$

$V = -CH_2-CO-NH-CHR-COOH$

et $Y = -R_3-SO_2R_2$

(R, $R_1$, $R_2$ et $R_3$ ayant la même signification que précédemment), en ce qu'au cours de la première étape, on procède à la carboxyméthylation, en ce qu'au cours d'une deuxième étape, on fixe les amines appropriées ou le chlorure de benzyle, en ce qu'au cours d'une troisième étape, on procède à la chlorosulfonation, en ce qu'au cours d'une quatrième étape, on fixe des acides aminés.

16. Application des produits selon les Revendications 1 à 6 en tant que médicament à action anticoagulante.

## Patentansprüche

1. Produkte mit antikoagulierenden Eigenschaften, dadurch gekennzeichnet, daß sie aus nicht vernetzten löslichen Polymeren, Homopolymeren oder Copolymeren bestehen, die aus Polystyrol und den Polysacchariden ausgewählt werden, die in ihrer Kette substituierbare Gruppen tragen, an die in statistischer Weise Guppen X und/oder Y und/oder V gebunden sind, worin

X die Gruppe $-SO_3R_1$ oder $-R_3-SO_3R_1$ bezeichnet,
$R_1$ ein Wasserstoffatom oder ein physiologisch verträgliches Metall darstellt,
$R_3$ eine Gruppe $-CH_2-CO-NH-R_4-$, worin $R_4$ einen Ethylen-, Arylen- oder Alkylarylen-Rest, der substituiert ist oder nicht,

oder $-CH_2$ 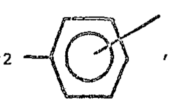 , das substituiert ist oder nicht, bedeutet,

Y die Gruppe $-SO_2-R_2$ oder $-R_3-SO_2-R_2$ bedeutet,
$R_2$ den Rest einer beliebigen Aminosäure im Falle der Polysaccharide bedeutet und im Falle von Polystyrol ausgewählt ist aus der Gruppe von Methionin, Cystein, Cysteinsäure, Prolin, Hydroxyprolin, Serin, Tyrosin, Benzyloxycarbonyl-lysin, tert.-Butyloxycarbonyl-lysin, $\varepsilon$-Aminocapronsäure, $\beta$-Alanin, $\gamma$-Amino-n-buttersaure, $\delta$-Amino-n-Valeriansäure, die substituiert sind oder nicht, wobei diese Aminosäure an die $-SO_2-$ Brücke durch ihre Aminfunktion gebunden ist, und
V die Gruppe $-CH_2-CO-NH-CHR-COOH$ bedeutet,
R die Seitenkette einer Aminosäure darstellt, wobei:

a) wenn X $-SO_3R_1$ ist, dieses von Y und/oder V begleitet sein muß und
b) V immer von X und/oder Y begleitet wird.

2. Produkte nach Anspruch 1, dadurch gekennzeichnet, daß im Falle der Polysaccharide die Aminosäuren aus solchen ausgewählt sind, die mindestens eine freie Carbonsäurefunktion enthalten.

3. Produkte nach Anspruch 1, dadurch gekennzeichnet, daß die Aminosäure eine Aminodicarbonsäure ist.

4. Produkte nach einem der Ansprüche 1 bis 2, dadurch gekennzeichnet, daß wenn die Aminosäuren mehrere Aminfunktionen enthalten, sämtliche, mit Ausnahme von einer, durch eine physiologisch brauchbare elektronenanziehende Gruppe blockiert sind.

5. Produkte nach Anspruch 4, dadurch gekennzeichnet, daß die elektronenanziehende Gruppe vorteilhaft durch eine Benzyloxycarbonyl- oder tert.-Butyloxycarbonylgruppe gebildet wird.

6. Produkte nach Anspruch 1, dadurch gekennzeichnet, daß die Polymeren Dextrane sind.

7. Verfahren zur Herstellung der Produkte nach einem der Ansprüche 1 bis 6, dadurch gekennzeichen, daß wenn man die Produkte erhalten will, worin

$X{=}{-}SO_3R_1$

$Y{=}{-}SO_2{-}R_2$

und $V{=}$Null

(wobei $R_1$ und $R_2$ die gleiche Bedeutung wie vorstehend aufweisen) man im Verlauf einer ersten Stufe ein chlorsulfoniertes Polymeres POL—$SO_2Cl$ durch Reaktion von Chlorsulfonsäure mit dem Polymeren in einem geeigneten Lösungsmittel herstellt und daß man im Verlauf einer zweiten Stufe die —$SO_2Cl$ Gruppen in —$SO_3Na$ Gruppen und in Gruppen —$SO_2AA$ (worin AA eine Aminosäure bedeutet) durch Reaktion mit einer geeigneten Menge einer Aminosäure in basischem Milieu umwandelt.

8. Verfahren nach Anspruch 7, dadurch gekennzeichnet, daß die Sulfonierung des nicht vernetzten Polymeren in einem organischen Milieu erfolgt, das vorteilhaft aus einem chlorierten Lösungsmittel gebildet wird, das Polymere in Nitromethan ausgefällt wird und die Reaktion mit der Aminosäure in einem Milieu, das das Gemisch Wasser-Dioxan enthält, durchgeführt wird.

9. Verfahren nach Anspruch 3, dadurch gekennzeichnet, daß das Sulfonierungsmilieu durch Dichlormethan gebildet wird.

10. Verfahren nach Anspruch 8, dadurch gekennzeichnet, daß man die Verunreinigungen vor der Bildung der Aminosäure durch Dialyse gegen Wasser entfernt, und falls gewünscht das reine Endprodukt durch Lyophilisieren gewinnt.

11. Verfahren zur Herstellung der Produkte nach einem der Ansprüche 1 bis 6, dadurch gekennzeichnet, daß wenn man Produkte erhalten will, worin

$X{=}{-}R_3{-}SO_3R_1$

$V{=}{-}CH_2{-}CO{-}NH{-}CHR{-}COOH$

und Y Null ist

(worin R, $R_1$ und $R_3$ die gleiche Bedeutung wie vorstehend aufweisen), man im Verlauf einer ersten Stufe die carboxymethylierten Derivate der Polymeren herstellt, daß man im Verlauf der zweiten Stufe die geeigneten Amine oder Benzylchlorid bindet, daß man im Verlauf der dritten Stufe die Aminosäuren bindet und daß man die Sulfonierung im Verlauf der vierten Stufe durchführt.

12. Verfahren nach Anspruch 11, dadurch gekennzeichnet, daß man die Kupplung der Amine und/oder Aminosäuren an die carboxymethylierten Derivate der Polymeren mit N-Ethoxycarbonyl-2-ethoxy-1,2-dihydrochinolein (EEDQ) bewirkt.

13. Verfahren nach Anspruch 11, dadurch gekennzeichnet, daß die Sulfonierungsstufe mehrfach wiederholt wird, wenn der Gehalt an Sulfonatgruppen erhöht werden soll.

14. Verfahren nach Anspruch 11, dadurch gekennzeichnet, daß das Endprodukt durch Ultrafiltration unter Druck gereinigt wird.

15. Verfahren zur Herstellung der Produkte nach einem der Ansprüche 1 bis 6, dadurch gekennzeichnet, daß wenn man Produkte erhalten will worin

$X{=}{-}R_3{-}SO_3R1$

$V{=}{-}CH_2{-}CO{-}NH{-}CHR{-}COOH$

und $Y{=}{-}R_3{-}SO_2R_2$

(worin R, $R_1$, $R_2$ und $R_3$ die gleiche Bedeutung wie vorstehend aufweisen) man im Verlauf der ersten Stufe die Carboxymethylierung durchführt und daß man im Verlauf einer zweiten Stufe die geeigneten Amine oder das Benzylchlorid bindet, daß man im Verlauf einer dritten Stufe die Chlorsulfonierung durchführt, daß man im Verlauf einer vierten Stufe die Aminosäuren bindet.

16. Verwendung der Produkte nach den Ansprüche 1 bis 6 als Arzneimittel mit antikoagulierenden Wirkung.

## Claims

1. Products endowed with anticoagulant properties, characterized in that they are constituted by uncrosslinked and soluble polymers, homopolymers or copolymers selected from among polystyrene and polysaccharides, comprising in their chains substitutable groups to which are fixed statistically, groups X and/or Y and/or V, where

X denotes the group $-SO_3R_1$ or $-R_3-SO_3R_1$
$R_1$ being a hydrogen atom or a physiologically compatible metal,
$R_3$ being a $-CH_2-CO-NH-R_4-$ group in which $R_4$ represents a substituted or unsubstituted ethylene, arylene or alkylarylene radical, or substituted or unsubstituted

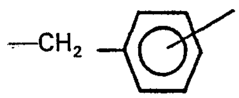

Y denotes the group $-SO_2-R_2$ or $-R_3-SO_2-R_2$,
$R_2$ being the residue of any amino acid in the case of polysaccharides and selected in the case of polystyrene from the group which comprises methionine, cysteine, cysteic acid, proline, hydroxyproline, serine, tyrosine, benzyloxycarbonyl-lysine, tertiobutyloxycarbonyl-lysine, ε-aminocaproic acid, β-alanine, γ-amino-n-butyric acid, δ-amino-n-valeric acid, whether substituted or not, which amino acid is joined to the $-SO_2-$ bridge through its amino function, and
V denotes the group $-CH_2-CO-NH-CHR-COOH$,
R being the side chain of an amino acid,

it being well understood that:

a) if X is $-SO_3R_1$, it is obligatorily accompanied by Y and/or V, and
b) V is always accompanied by X and/or Y.

2. Products according to Claim 1, characterized in that, in the case of polysaccharides, the amino acids are selected from among those which comprise at least one free carboxylic function.

3. Products according to Claim 1, characterized in that the amino acid is dicarboxylic amino acid.

4. Products according to any one of the Claims 1 to 2, characterized in that when the amino acids possess several amino functions, all except one must be blocked by a physiologically acceptable electroattractive group.

5. Products according to Claim 4, characterized in that the electroattractive group is advantageously constituted by a benzyloxycarbonyl or tertiobutyloxycarbonyl.

6. Products according to Claim 1, characterized in that the polymers are dextrans.

7. Process for the preparation of products according to any one of Claims 1 to 6, characterized—when it is desired to obtain the products in which

$$X = -SO_3R_1$$

$$Y = -SO_2-R_2$$

and V=nil

($R_1$ and $R_2$ having the same meaning as previously)—in that in the course of the first step, a chlorosulfonated polymer $POL-SO_2Cl$ is prepared by the reaction of chlorosulfonic acid on the polymer, in a suitable solvent, and in that in the course of the second step, the $-SO_2Cl$ groups are converted into $-SO_3Na$ groups and into $-SO_2AA$ groups (where AA represents an amino acid) by reaction with a suitable amount of an amino acid in a basic medium.

8. Process according to Claim 7, characterized in that the sulfonation of the uncrosslinked polymer takes place in an organic medium advantageously constituted by a chlorinated solvent, the polymer is precipitated in nitromethane and the reaction with the amino acid takes place in a medium containing water-dioxane mixture.

9. Process according to Claim 8, characterized in that the sulfonation medium is constituted by dichloromethane.

10. Process according to Claim 8, characterized in that the impurities are removed after fixing the amino acid, by dialysis against water and there is recovered, if desired, the pure finished product by freeze-drying.

11. Process for the preparation of products according to any one of Claims 1 to 6, characterized in that when it is desired to obtain the product in which:

$$X = -R_3-SO_3R_1$$

$$V = -CH_2-CO-NH-CHR-COOH$$

and Y is nil

(R, $R_1$ and $R_3$ having the same meaning as previously), in that there is prepared, in the course of the first step, the carboxymethylated derivatives of the polymers, in that there is fixed, in the course of the second step, the appropriate amines or benzyl chloride, in that the amino acids are fixed in the course of the third step and in that sulfonation follows in the course of the fourth step.

12. Process according to Claim 11, characterized in that the coupling of the amines and/or of the amino acids to the carboxymethylated derivatives of the polymers is carried out by means of N-ethoxycarbonyl-2-ethoxy 1,2-dihydroquinoleine (EEDQ).

13. Process according to Claim 11, characterized in that the sulfonation step is repeated several times if it is desired to increase the ratio of sulfonate groups.

14. Process according to Claim 11, characterized in that the finished product is purified by ultrafiltration under pressure.

15. Process for the preparation of the products according to any one of Claims 1 to 6, characterized, when it is desired to obtain the products in which:

$$X = -R_3-SO_3R_1$$

$$V = -CH_2-CO-NH-CHR-COOH$$

and $Y = -R_3-SO_2R_2$

(R, $R_1$, $R_2$ and $R_3$ having the same meaning as previously), in that in the course of the first step, carboxymethylation follows, in that in the course of the second step, the appropriate amines or benzyl chloride are fixed, in that in the course of the third step, chlorosulfonation follows, in that in the course of the fourth step, the amino acids are fixed.

16. Use of the products according to Claims 1 to 6, as a medicament with anticoagulant effect.

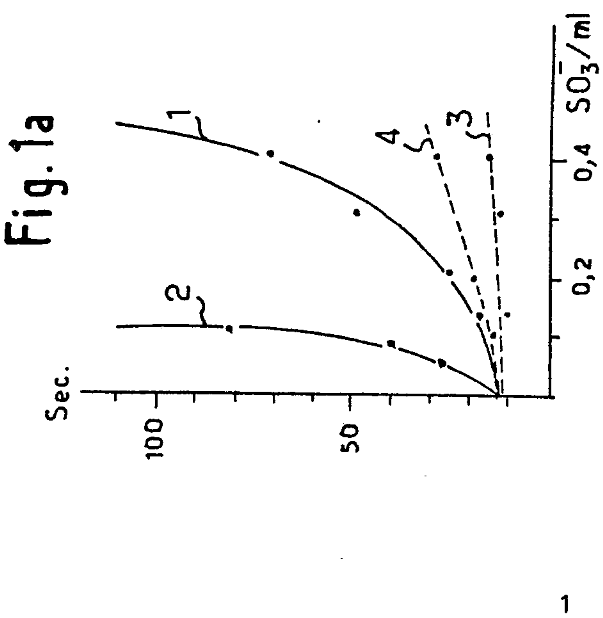


Fig. 1a

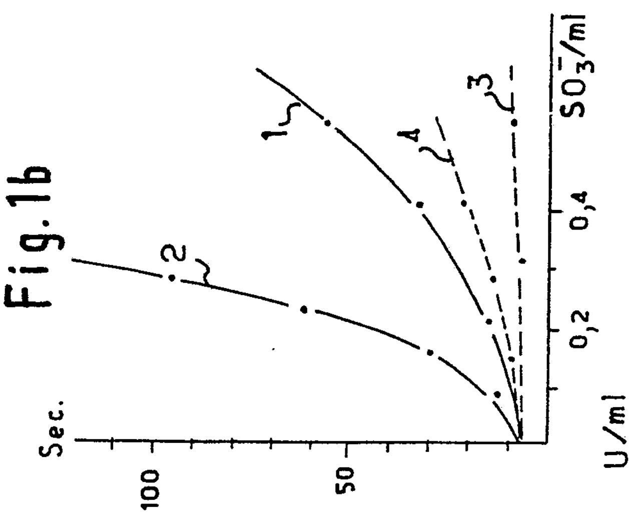


Fig. 1b

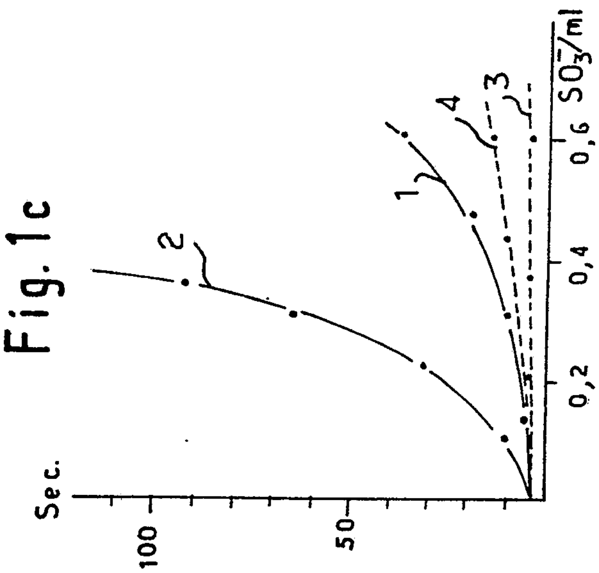


Fig. 1c

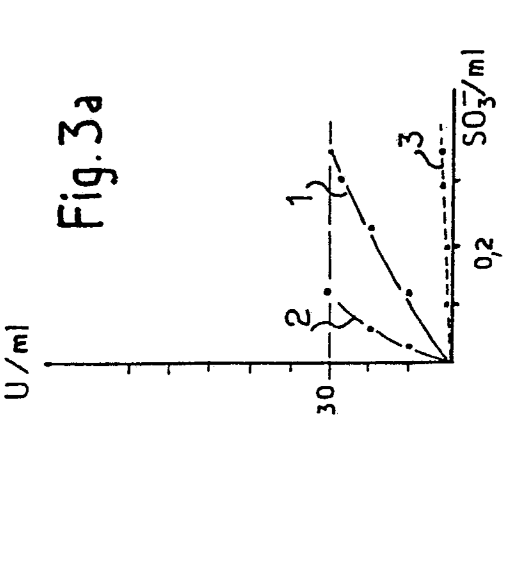


Fig. 3a

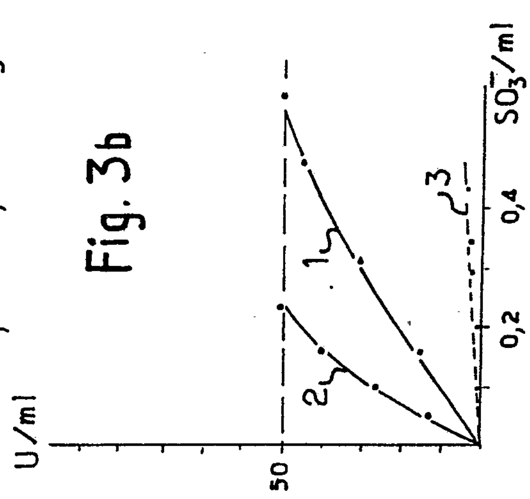


Fig. 3b

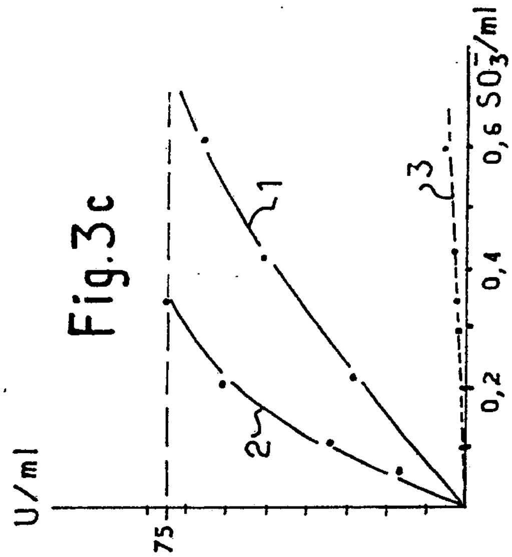


Fig. 3c

Fig. 11b

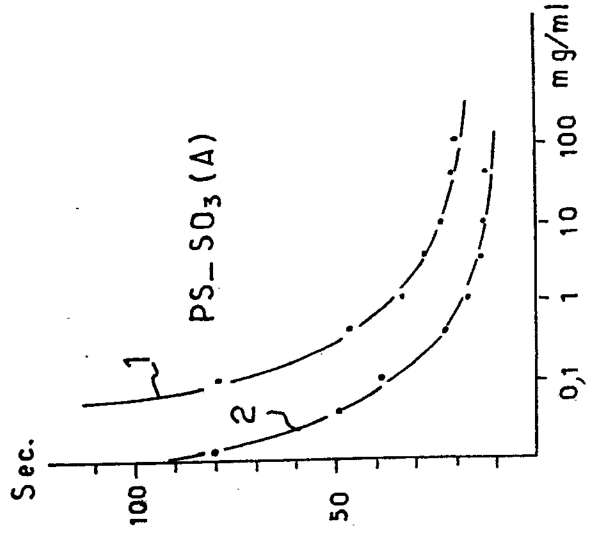


Fig. 11a

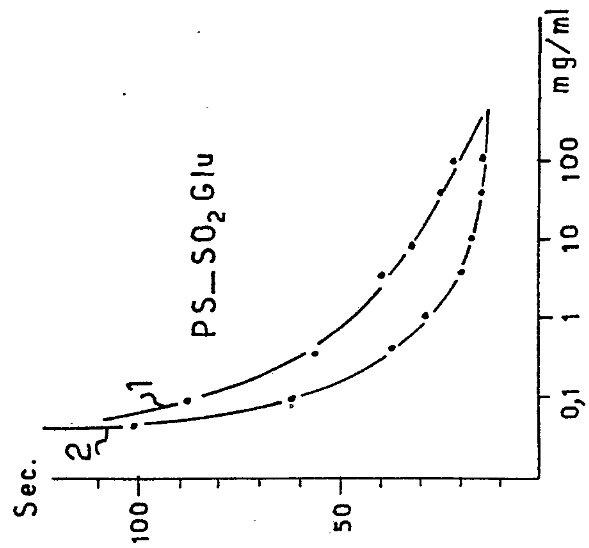


Fig. 2

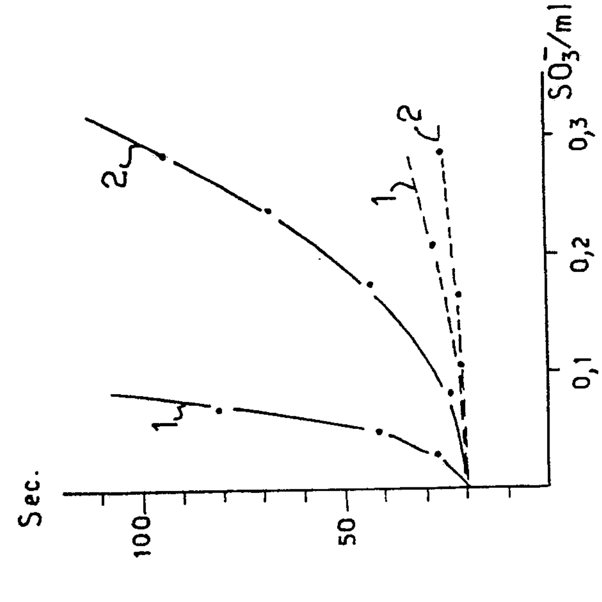

Fig. 4

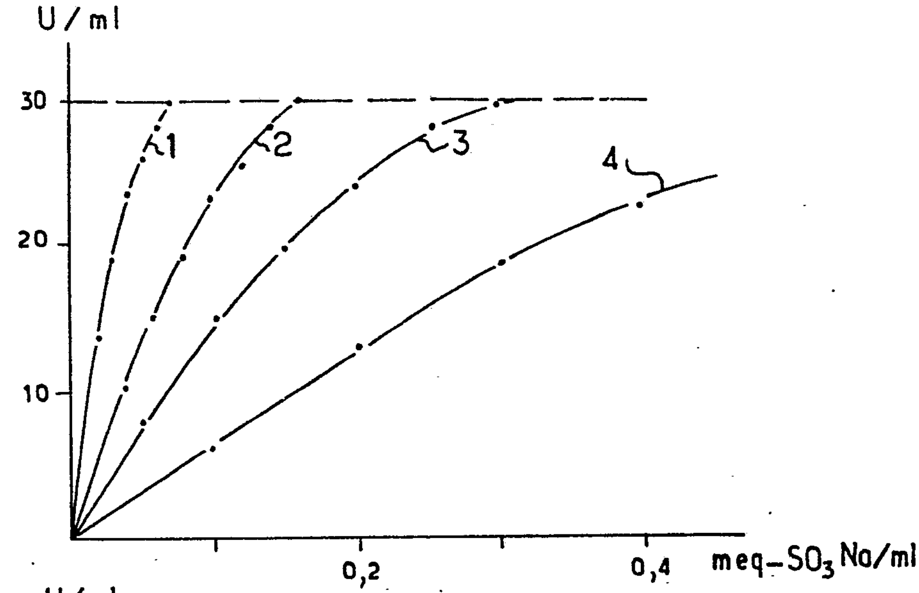


Fig. 5

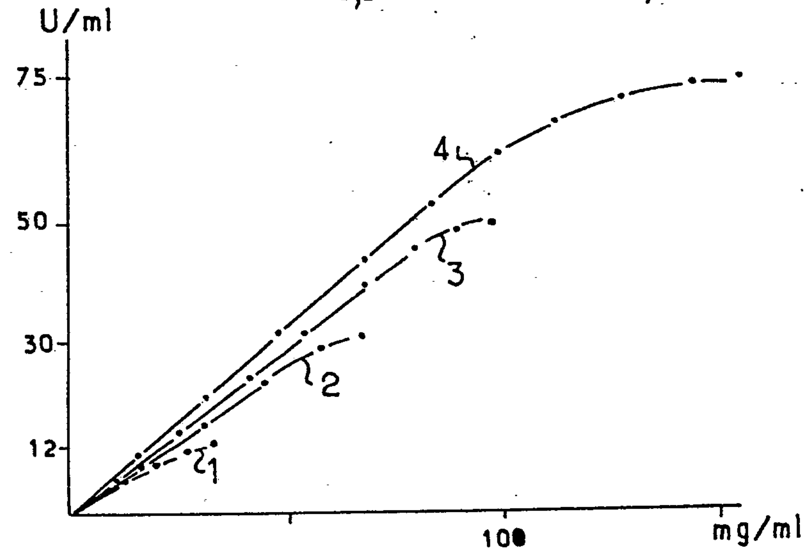


Fig. 6

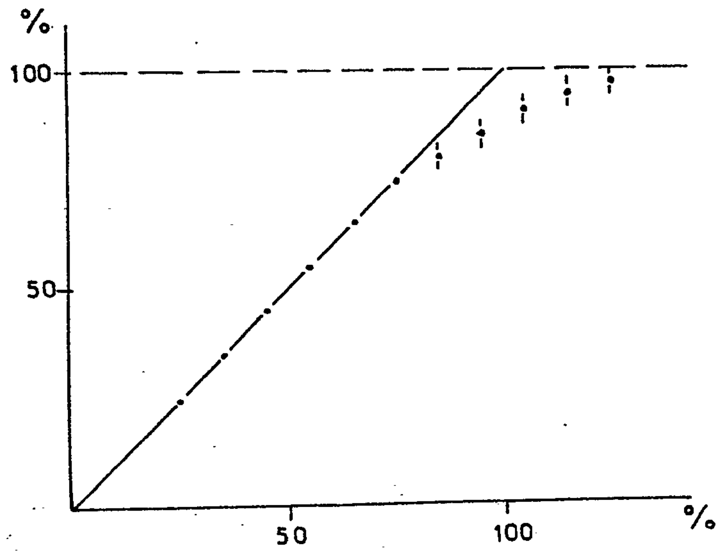

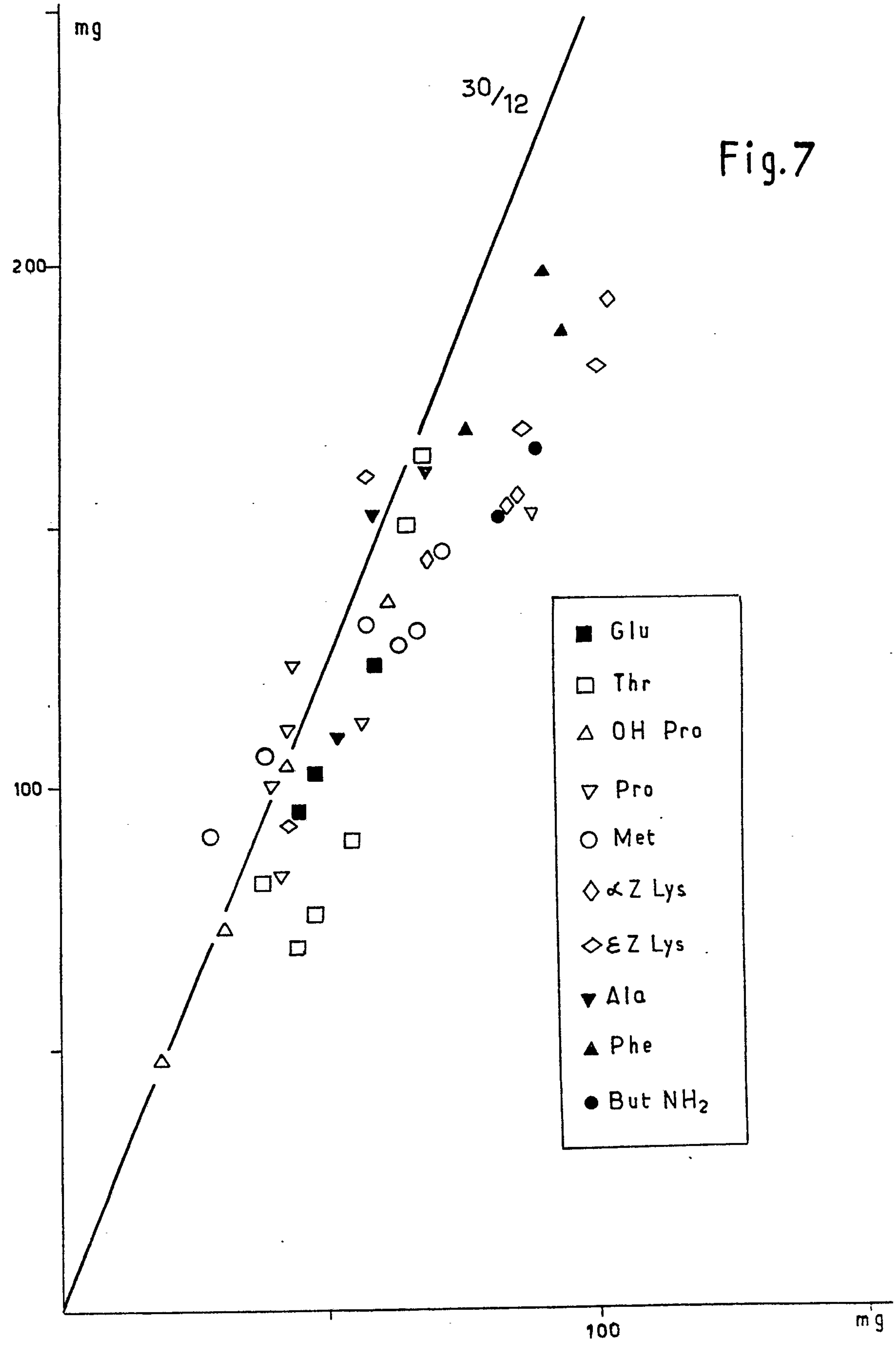
mg
30/12
200
100
100
mg
Glu
Thr
OH Pro
Pro
Met
α Z Lys
ε Z Lys
Ala
Phe
But NH2

Fig.7

Fig. 8

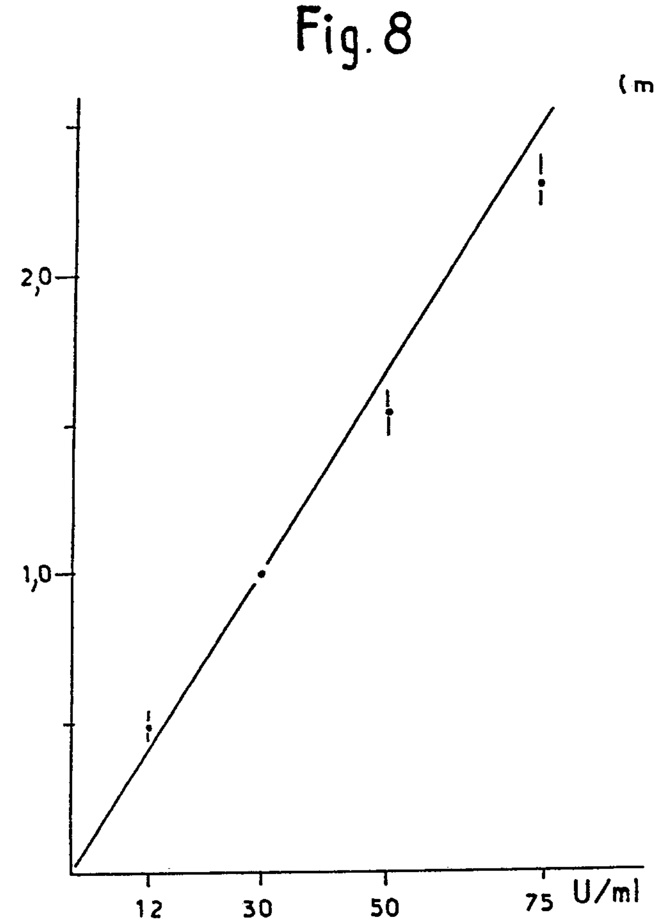


Fig. 10

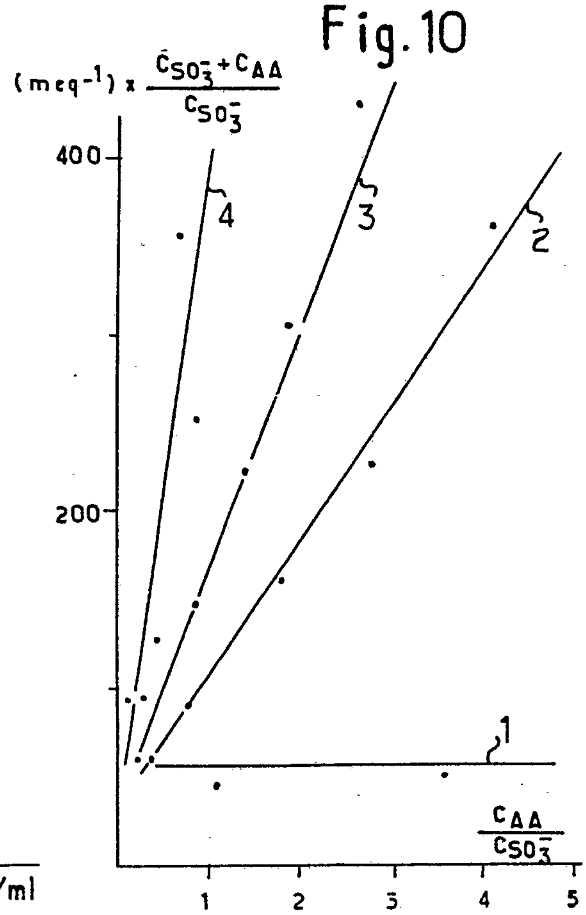


Fig. 9